# EUROPEAN PATENT SPECIFICATION

(11) **EP 1 374 926 B1**
(45) Date of publication and mention of the grant of the patent: **15.11.2006**
(21) Application number: 02425426.0
(22) Date of filing: 28.06.2002
(51) Int. Cl.: A61M 1/00, A61M 39/06

(54) **Self-sealing valve for medical use**
Selbstverschliessendes Ventil für medizinische Zwecke
Soupape auto-étanche à usage médical

(43) Date of publication of application: 02.01.2004
(73) Proprietor: Bioservice S.p.A., 46025 Poggio Rusco (MN) (IT)
(72) Inventor: Zucchi, Giuseppe, 41039 San Possidionio (Modena) (IT); Baraldi, Massimo, 41036 Medolla (MO) (IT); Musicco, Gian Franco, 25124 Brescia (BS) (IT)
(74) Representative: Trupiano, Federica

(56) References cited:
- US-A- 5 059 186
- US-A- 5 403 284
- US-A- 5 591 137
- US-A- 5 921 968
- US-A1- 2002 010 436

## Description

The present invention relates to a self-sealing valve for medical use. In particular, the present invention relates to a self-sealing valve for use in devices for removal of drainage liquid and more in particular for a device for carrying out thoracentesis.

Thoracentesis is a method for removal of transudate or exudate present in the pleural cavity following upon pleural affections.

According to the prior art, for said purpose a catheter is introduced into the pleural cavity through the wall of the thorax by means of a needle that extends along the catheter. The introduction of the needle must be carried out with great care to prevent air from entering into the pleural cavity, and to prevent the needle from perforating the internal wall of the pleura and approaching the lung too closely, so as to eliminate any possible risk of perforation of the lung with the needle. The entry of air into the pleural cavity or the perforation of the lung would cause collapse of the latter. In more recent embodiments, the needle is withdrawn after perforating the first wall, leaving the catheter in position, connected to suction means using one or more valves.

The US patent N° 5,921,968 discloses a hemostasis valve including a tubular body, a compressible seal, a quick release mechanism, a plunger biasing means and a rotatable end cap. The compressible seal is cylindrical in shape and an annular rib is integrally formed on its interior surface. This annular rib allows an elongates instrument (cateter), accessing lumen through passageway, to be repositioned or removed without releasing substantially all the compressive force acting on said sealing means. The plunger is used to evenly distribute the compressive forces onto the seal applied by means of threading the end cap. The quick release mechanism is configured to selectively move between an inactivated position (which allows the seal to be in a compressed position) and an activated position (which causes the seal to be in a released position with substantially no compressive force acting on the seal). A quick release lever can slide longitudinally relative to the body between a first position and a second position causing quick-release mechanism to slide between said inactivated position and activated position.

Consequently said lever allows the selective releasing and restoring the desired amount of compressive force applied by said end cap on the seal.

US-A-5,403,284 discloses a self-sealing valve designed so that the re-insertion of the needle into the valve after it has been withdrawn is prevented. The valve comprises a valve body, a mobile element, a biasing means and a sealing means. When the needle, passing through the valve, is removed, the sealing means sealably fits against the housing inner seat to prevent air flow therethrough This is obtained by the particular geometrical formation of bom said inner seat and sealing means. In particularly, when the sealing means has a spherical body, the seat has an accordingly shaped counterpart form. When the needle is removed from the valve, the biasing means urges the stealing means to slide sideways within the body so that the passageway in the sealing means and in the lumen become misaligned.

The European patent EP-0835152 describes a self-sealing valve used in a device for thoracentesis. The valve comprises a body wherein a mobile plunger is positioned, which is set in motion by a spring following upon withdrawal of the needle from the catheter and from the body of the valve. Said motion of the plunger pushes a ball into a seat defined by the annular and inclined surface of the body of the valve. Subsequently, upon withdrawal of the needle from the pleural cavity, the valve prevents the needle from being inserted again through the valve and the catheter.

The European patent EP-0943356 relates to a device for thoracentesis and for removal of drainage liquid, which comprises a syringe, a catheter, a needle that can be removed from the catheter, a first valve, fixed on the catheter for deviation of the liquid removed, and a self-sealing valve, connected to the first valve by means of a Luer-lock connection. The first valve must be switched manually into a second position to enable removal of the drainage liquid from the pleural cavity or abdominal cavity in a vacuum environment. The self-sealing valve corresponds to the valve described in the European patent EP-0835152. The use of a Luer-lock connection enables fast and simple disassembly of the device and hence the use of the catheter for a long period of time for removing drainage liquid.

The device for thoracentesis proposed in the European patent EP-0662010 is made up of a syringe, a catheter, and a valve assembly fixed on the catheter. The body of the valve has a central hole in a position coaxial to the catheter for receiving the thoracentesis needle and a side draining portion. When the needle is withdrawn from the catheter and from the valve body, the side draining portion is connected internally to the catheter to carry away the drainage flow from the pleural cavity. The central hole is sealed automatically when the needle is removed from the valve body. Said automatic seal is obtained with a ball that is pushed by a biasing means activated by a spring following upon removal of the needle. In the valve body, there are provided also elastic means, such as, for example, elastic sheaths, O-rings, and thin cylinders, positioned adjacent to the seat of the ball in order to block the central hole in the direction of the catheter.

The above known valves, used in devices for thoracentesis, have a relatively complex structure, which is expensive to be produced and is subjected to malfunctioning. Furthermore, the use of the valve described in the European patent EP-0943356 in devices for thoracentesis, also requires a "first valve" for removal of the drainage liquid from the pleural cavity. In addition, said valve, as also the valve according to the European patent EP-0662010, frequently does not ensure a secure and hermetic closure of the valve after withdrawal of the needle from the valve body.

Consequently, the purpose of the present invention is to provide a valve which has a simple, reliable and economical structure, which ensures a secure hermetic seal, and which prevents re-introduction of the needle into the catheter after it has been removed therefrom.

Said purpose is achieved according to the present invention with a self-sealing valve characterized according to Claim 1.

The self-sealing valve according to the present invention is usable for removal of liquid, such as, for example, drainage liquid, from different parts of the body; in particular it is suited for use in devices for thoracentesis.

The part of the mobile element that is in the direction of the sealing means, i.e., the head of the mobile element, is shaped and has at least one inclined plane; preferably it has two symmetrical inclined planes.

The self-sealing valve for removal of liquid according to the present invention is formed in such a way that it can receive a needle that extends along the valve body when the mobile element is in the first position, hence when the valve is open.

The body of the valve has a front end and a rear end. The rear end is closed by a membrane covering made of a flexible material, such as, for example, silicone. Said closure or covering membrane is perforated when the thoracentesis needle is inserted into the catheter through the opening of the valve body.

The valve body is made up of a front portion and a rear portion fixed together to form a single body. The front portion forms the front end of the valve body, where the seat of the sealing means is positioned.

The rear portion of the valve comprises a seat for the biasing means for the mobile element that deforms the sealing means. The biasing means is preferably a spring.

The sealing means is preferably a tube made of a soft and flexible plastic material. The said plastic material has preferably a Shore A hardness of between 20 and 80 and is deformable under the action of the mobile element biased by the spring or other biasing means.

The tube is housed in a sealed way in a seat in the front position of the valve body.

The tube has an opening that traverses it and that has preferably dimensions designed for enabling the needle to slide through the tube without effort and without leaving too much play between the needle and the tube.

The head of the mobile element closes the opening of the sealing means hermetically when the mobile element is in the second closed valve position, following upon withdrawal of the needle from the catheter and from the valve body.

The front end of the valve body has preferably an end portion for accommodating a catheter and has a side portion for removal of the drainage liquid.

The self-sealing valve according to the present invention is preferably made using biocompatible plastic materials, such as, for example, polycarbonate, ABS, PVC, etc.

Further characteristics and advantages of the present invention will be evident to persons skilled in the relevant art from the following description of the preferred embodiments that exemplify the best mode of embodiment of the present invention, and in which:
Figure 1 is a cross-sectional view along the principal longitudinal axis of a preferred embodiment of the self-sealing valve according to the present invention, without needle, catheter, or spring;
Figure 2 is a cross-sectional view taken along the principal longitudinal axis of a device for thoracentesis in the open condition, with needle; and
Figure 3 illustrates the device for thoracentesis according to Figure 2 without needle, with closed valve.

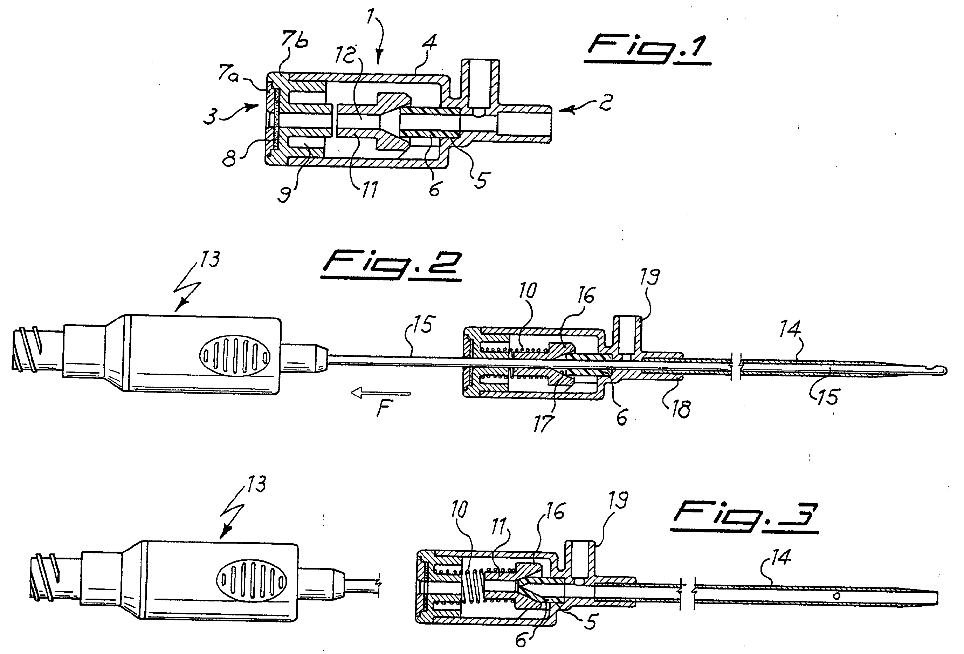

The preferred embodiment of the self-sealing valve according to the present invention represented in Figure 1 comprises a valve body 1 having a front end 2, and a rear end 3. In the front end 2 of the valve body there is formed externally a seat 18 for the catheter 14 (Figs. 2-3) and internally a seat 5 for housing the end of the sealing means. The sealing means is a tube made of deformable material; preferably the tube 6 is made of elastomeric material, such as silicone, poly-isoprene, etc. As mentioned above, the sealing tube 6 has an internal diameter with dimensions such as to enable housing of the needle 15 in a freely slidable way (Fig. 2). The thickness of the walls of the tube is in general between 0.5 mm and 3.0 mm and is such as to enable the tube to deform only after the needle 15 has been removed from the valve 1. The length of the tube is such as to enable its closing by compression and deformation of at least the rear end of the tube, namely, of the end engaged with the inclined plane or inclined planes of the mobile element 16.

In general, the ratio L/D between the length L and the diameter D ranges between 1 and 10.

The rear end 3 of the valve body 1 is closed by a membrane 8 made of a flexible material, positioned in the rear portion 7a and 7b of the valve body. The front portion 4 and the rear portion 7a and 7b of the valve body 1 are fixed together after assembly to form a single body that cannot be opened or dismantled without rendering the valve unusable.

The rear portion 7b comprises a seat 9 for the spring 10 that functions as biasing means for the mobile element, as represented in Figs. 2 and 3. One end of the spring is fixed in the seat 9 formed in the annular recess of the rear portion 7b, whilst the other end of the spring engages the rear end of the mobile element 11. The mobile element 11 is provided with a through hole 12 that is coaxial to the valves, for introduction of a needle 15 (Fig. 2). The seat 9 also has the function of supporting and aligning the needle 15 with the opening present in the front part 4 of the valve 1.

Figure 2 represents the self-sealing valve as an element of a device for thoracentesis, which further comprises a catheter 14 and a needle 15, fixed at one end to a grip 13, the said needle 15 traversing the valve body 1 and the catheter 14. As specified above, the needle traverses also the spring 10, the mobile element 16, and the sealing tube 6.

The needles preferably used are the ones known from the prior art (for example the Veress needle), which are designed for preventing the perforation of the wall of the pleural cavity in the direction of the lung.

Figure 2 represents the mobile element 11 of the valve in its first position in the valve body, namely when the valve is open. The head of the mobile element 16 is shaped, and is formed by at least one and preferably two inclined planes 17, which form a central opening having the shape of a truncated cone for housing the end of the sealing tube 6. The catheter 14 is mounted in a seat provided in the end portion 18 of the valve. Said end portion 18 has a front side connection for deviation of the flow of liquid removed through the catheter from the pleural cavity once the needle 15 has been withdrawn, as is explained in greater detail in what follows. The seat on the portion of the valve 18 has substantially the same internal diameter as the external diameter of the catheter 14.

When the needle 15 is inserted into the catheter 14, the drainage liquid does not pass through the hole of the side connection 19, but flows through the entire needle 15 and the grip 13, and is then suctioned in a known way. In this condition, the needle 15 supports the tube 6 and prevents the tube from being deformed under the action of the spring 10 and of the mobile element 16.

Figure 3 represents the self-sealing valve in use in a device for thoracentesis, in which the needle has been withdrawn from the catheter 14 and from the valve body 1 in the direction of the arrow F of Fig. 2. Following upon withdrawal of the needle from the valve, the mobile element 11, biased towards the end portion of the valve of the spring 10, displaces from the first position (i.e., the open-valve position) into a second position (the closed-valve position), in which its shaped head 16 with the opening shaped like a truncated cone 17 presses and closes simultaneously the tube 6, which is deformed in so far as it is no longer supported by the needle.

As may be seen in Fig. 3, since the needle 15, which previously kept all the components of the valve according to the invention aligned, is no longer present when the mobile element 11 is in the second position in the valve body, the various elements - spring 10, mobile element 16 and tube 6 - are misaligned with respect to one another and with the openings present in the rear wall 7a+7b and front wall 4 of the valve. In this way, the re-insertion of the needle into the catheter through the valve is prevented. The front end of the deformable tube 6 is housed in a sealed way in its seat 5, whilst the rear end is closed by deformation by the shaped head of the mobile element 16, thus obtaining a hermetic closure of the valve. The drainage liquid removed from the pleural cavity through the catheter 14 of the device for thoracentesis is at this point deviated through the side connection 19 of the valve according to the present invention and removed in a known way.

## Claims

1. A self-sealing valve for medical use with a drainage syringe comprising:
- a valve body (1) having a front end (2) anda rear end (3), and comprising a rear portion (7b), and being provided with an opening;
- a mobile element(11) that can move between a first position and a second position in the valve body (1),
- biasing means (10) for said mobile element (11) to bias said element in the direction of the front end (2) of the valve body (1), one end of said biasing means is fixed in a seat (9) formed in said valve body rear portion (7b), whilst the other end of the biasing means engages the rear end of the mobile element (11) to bias it towards said valve body front end (2);
- sealing means (6) engaged with said mobile element (11) and said front opening (2) and located between them, and said sealing means (6) housing a needle portion (15) of said drainage syringe when said mobile element (11) is in the aforementioned first position; said sealing means is provided for the hermetic closure of the front opening (2) of the valve body when the mobile element (11) is pushed into the second position;
**characterised in that** the sealing means (6) comprises a tubular element made of a deformable material which is deformed in so far as it is no longer supported by the needle and closed by said mobile element after the needle (15) has been removed from the valve (1).

2. The valve according to Claim 1, **characterized in that** said mobile element (11) comprises a through hole and is aligned with said front opening (2) and said rear opening (3) and with said tubular sealing element (6).

3. The valve according to Claim 1 or 2, **characterized in that** the part of the mobile element (11) that engages said tubular sealing element (6), is shaped for closing said tubular element (6) by deformation.

4. The valve according to Claim 3, **characterized in that** said shaped portion of the mobile element (11) comprises two inclined planes (17) diverging from said through hole outwards.

5. The valve according to any one of the preceding claims
**characterized in that**, in the open-valve condition, said needle (15) traverses said valve and is coaxially housed in said rear and front portions, in said biasing means (10), in said mobile element (11), and in said tubular sealing element (6).

6. The valve according to any one of the preceding claims, **characterized in that** at least said mobile element (11), when it is displaced from the first position to the second position following upon withdrawal of the needle (15), is misaligned with respect to said front opening (2) and said rear opening (3).

7. The valve according to any one of the preceding claims, **characterized in that** said front end (2) of the valve body (1) has an end portion (18) for accommodating a catheter (14) and has a side portion (19) for removal of liquid.

8. The valve according to any one of the preceding claims, **characterized in that** in the said front end (2) of the valve body (1), there is formed internally a seat (5) for said tubular sealing element (6).

9. The valve according to any one of the preceding claims, **characterized in that** the valve body (1) has a front end (2) and a rear end (3), where the rear end (3) is closed by a covering made of a flexible material.

10. The valve according to any one of the preceding claims, **characterized in that** the rear portion (3) of the valve body (1) comprises a portion projecting internally, which functions as a seat for said biasing means and as a support and guide for said needle (15).

11. A device for thoracentesis, **characterized in that** it comprises a valve (1) according to any one of Claims 1 to 10.

## Patentansprüche

1. Selbstverschließendes Ventil für medizinische Zwecke mit einer Drainagespritze umfassend:
- ein Ventilgehäuse (1) mit einem vorderen Ende (2) und einem hinteren Ende (3) und umfassend ein hinteres Teil (7b) und ausgestattet mit einer Öffnung;
- ein mobiles Element (11), das sich zwischen einer ersten Position und einer zweiten Position im Ventilgehäuse (1) bewegen kann,
- Vorspannvorrichtung (10) für das mobile Element (11), um das Element in Richtung vorderes Ende (2) des Ventilgehäuses (1) vorzuspannen, wobei ein Ende der Vorspannvorrichtung in einem Sitz (9) befestigt ist, der sich im hinteren Teil (7b) des Ventilgehäuses befindet, wohingegen das andere Ende der Vorspannvorrichtung in das hintere Ende des mobilen Elements (11) eingreift, um es in Richtung vorderes Ende (2) des Ventilgehäuses zu spannen;
- Dichtvorrichtung (6), die in das mobile Element (11) und die vordere Öffnung (2) eingreift und sich dazwischen befindet, wobei die Dichtvorrichtung (6) ein Nadelteil (15) der Drainagespritze aufnimmt, wenn das mobile Element (11) sich in der vorgenannten ersten Position befindet; die Dichtvorrichtung wird für die hermetische Abdichtung der vorderen Öffnung (2) des Ventilgehäuses bereitgestellt, wenn das mobile Element (11) in die zweite Position gedrückt wird;
**dadurch gekennzeichnet, dass** die Dichtvorrichtung (6) ein aus einem formbaren Material hergestelltes röhrenförmiges Element umfasst, das insoweit verformt ist, dass es nicht mehr von der Nadel gehalten wird, und durch das mobile Element geschlossen wird, sobald die Nadel (15) aus dem Ventil (1) genommen wird.

2. Ventil nach Anspruch 1 **dadurch gekennzeichnet, dass** das mobile Element (11) eine Durchgangsbohrung umfasst und mit der vorderen Öffnung (2), der hinteren Öffnung (3) und dem röhrenförmigen Dichtelement (6) fluchtet.

3. Ventil nach Ansprüchen 1 oder 2 **dadurch gekennzeichnet, dass** das Teil des mobilen Elements (11), das mit dem röhrenförmigen Dichtelement (6) eingreift so geformt ist, dass ein Schließen des röhrenförmigen Elements (6) durch Deformation möglich ist.

4. Ventil nach Anspruch 3 **dadurch gekennzeichnet, dass** der geformte Teil des mobilen Elements (11) zwei geneigte Flächen (17) umfasst, die von der Durchgangsbohrung aus nach außen gerichtet sind.

5. Ventil nach einem der vorgenannten Ansprüche **dadurch gekennzeichnet, dass** bei geöffnetem Ventil die Nadel (15) das Ventil durchquert und koaxial im hinteren und vorderen Teil der Vorspannvorrichtung (10), im mobilen Element (11) und dem röhrenförmigen Element (6) aufgenommen wird.

6. Ventil nach einem der vorgenannten Ansprüche **dadurch gekennzeichnet, dass** mindestens das mobile Element (11), wenn es nach dem Zurückziehen der Nadel (15) von der ersten Position in die zweite Position gebracht wird, nicht fluchtend mit der vorderen Öffnung (2) und der hinteren Öffnung (3) ist.

7. Ventil nach einem der vorgenannten Ansprüche **dadurch gekennzeichnet, dass** das vordere Ende (2) des Ventilgehäuses (1) ein Endteil (18) zur Aufnahme eines Katheters (14) und ein Seitenteil (19) zur Abführung von Flüssigkeit besitzt.

8. Ventil nach einem der vorgenannten Ansprüche **dadurch gekennzeichnet, dass** im vorderen Ende (2) des Ventilgehäuses (1) ein intern geformter Sitz (5) für das röhrenförmige Dichtelement (6) vorhanden ist.

9. Ventil nach einem der vorgenannten Ansprüche **dadurch gekennzeichnet, dass** das Ventilgehäuse (1) ein vorderes Ende (2) und ein hinteres Ende (3) besitzt, wobei das hintere Ende (3) durch eine Abdeckung aus einem flexiblen Material geschlossen wird.

10. Ventil nach einem der vorgenannten Ansprüche **dadurch gekennzeichnet, dass** das Endteil (3) des Ventilgehäuses (1) ein nach innen vorstehendes Teil umfasst, das als Sitz für die Vorspannvorrichtung und als Stütze und Führung für die Nadel (15) dient.

11. Vorrichtung für Thorakozentese **dadurch gekennzeichnet, dass** es ein Ventil (1) nach einem der vorgenannten Ansprüche 1 bis 10 umfasst.

## Revendications

1. Soupape auto-étanche à usage médical avec seringue pour drainage comprenant :
- un corps de soupape (1) comportant une extrémité avant (2) et une extrémité arrière (3), et comprenant une partie arrière (7b), et pourvu d'une ouverture ;
- un élément mobile (11) pouvant se déplacer d'une première position à une seconde position dans le corps de la soupape (1),
- un moyen d'orientation (10) dudit élément mobile (11) pour orienter ledit élément en direction de l'extrémité avant (2) du corps de la soupape (1), une extrémité dudit moyen d'orientation étant fixée dans un support (9) formé dans ladite partie arrière du corps de soupape (7b), l'autre extrémité du moyen d'orientation s'engageant dans l'extrémité arrière de l'élément mobile (11) pour l'orienter en direction de ladite extrémité avant du corps de soupape (2) ;
- un moyen d'étanchéisation (6) engagé avec ledit élément mobile (11) et ladite ouverture avant (2) et situé entre eux, ledit moyen d'étanchéisation (6) abritant une partie d'aiguille (15) de ladite seringue pour drainage lorsque ledit élément mobile (11) se trouve dans la première position mentionnée précédemment ; ledit moyen d'étanchéisation est prévu pour fermer hermétiquement l'ouverture avant (2) du corps de soupape lorsque l'élément mobile (11) est poussé dans la seconde position ;
**caractérisé en ce que** le moyen d'étanchéisation (6) comprend un élément tubulaire fait d'un matériau déformable qui est déformé en ce sens qu'il n'est plus soutenu par l'aiguille et qui est fermé par ledit élément mobile après que l'aiguille (15) a été retirée de la soupape (1).

2. Soupape selon la revendication 1, **caractérisée en ce que** ledit élément mobile (11) comprend un conduit et est aligné avec ladite ouverture avant (2) et ladite ouverture arrière (3) et avec ledit élément tubulaire d'étanchéisation (6).

3. Soupape selon la revendication 1 ou 2, **caractérisée en ce que** la partie de l'élément mobile (11) qui s'engage dans ledit élément tubulaire d'étanchéisation (6) est profilée de façon à fermer ledit élément tubulaire (6) par déformation.

4. Soupape selon la revendication 3, **caractérisée en ce que** ladite partie profilée de l'élément mobile (11) comprend deux plans inclinés (17) divergeant dudit conduit vers l'extérieur.

5. Soupape selon l'une quelconque des revendications précédentes, **caractérisée en ce que**, lorsque la soupape est à l'état ouvert, ladite aiguille (15) traverse ladite soupape et est coaxialement abritée dans lesdites parties arrière et avant, dans ledit moyen d'orientation (10), dans ledit élément mobile (11), et dans ledit élément d'étanchéisation tubulaire (6).

6. Soupape selon l'une quelconque des revendications précédentes, **caractérisée en ce que** au moins ledit élément mobile (11), lorsqu'il est déplacé de la première position à la seconde position après retrait de l'aiguille (15), n'est pas aligné par rapport à ladite ouverture avant (2) et ladite ouverture arrière (3).

7. Soupape selon l'une quelconque des revendications précédentes, **caractérisée en ce que** ladite extrémité avant (2) du corps de soupape (1) dispose d'une partie avant (18) pour y adapter un cathéter (14) et d'une partie latérale (19) pour éliminer du liquide.

8. Soupape selon l'une quelconque des revendications précédentes, **caractérisée en ce que** dans ladite extrémité avant (2) du corps de soupape (1), il se forme à l'intérieur un support (5) pour ledit élément d'étanchéisation tubulaire (6).

9. Soupape selon l'une quelconque des revendications précédentes, **caractérisée en ce que** le corps de soupape (1) dispose d'une extrémité avant (2) et d'une extrémité arrière (3), où l'extrémité arrière (3) est fermée par un revêtement fait en matériau flexible.

10. Soupape selon l'une quelconque des revendications précédentes, **caractérisée en ce que** la partie arrière (3) du corps de soupape (1) comprend une partie se projetant à l'intérieur, qui fonctionne comme un support pour ledit moyen d'orientation et comme un support et un guide pour ladite aiguille (15).

11. Dispositif pour thoracentèse, **caractérisé en ce qu'**il comprend une soupape (1) selon l'une quelconque des revendications 1 à 10.
